(19) Europäisches Patentamt
European Patent Office
Office européen des brevets

(11) Numéro de publication : **0 001 727**
**B1**

(12) # FASCICULE DE BREVET EUROPÉEN

(45) Date de publication du fascicule du brevet : **11.11.81**

(51) Int. Cl.³ : **C 07 D277/48**, A 61 K 31/425

(21) Numéro de dépôt : **78400112.5**

(22) Date de dépôt : **26.09.78**

(54) **Nouveaux dérivés du phényl-4-(thrichloro-2',2',2'-éthoxycarboxamido)-2-thiazole utiles comme médicaments, compositions pharmaceutiques les contenant et leur procédé de préparation.**

(30) Priorité : **19.10.77 FR 7731687**

(43) Date de publication de la demande : **02.05.79 (Bulletin 79/09)**

(45) Mention de la délivrance du brevet : **11.11.81 Bulletin 81/45**

(84) Etats contractants désignés : **BE CH DE FR GB LU NL SE**

(56) Documents cités :
**DE - A - 2 132 431**
**US - A - 3 499 910**

**CHEMICAL ABSTRACTS, vol. 38, n° 19, 10 octobre 1944, Columbus, Ohio, USA**
**FRANCO LANFRANCHI et al. « Nicotinylaminothiazoles I. 2-Nicotinylamino-4-methylthiazole and 4-phenylthiazole »**
**CHEMICAL ABSTRACTS, vol. 53, n° 11, 10 juin 1959, Columbus, Ohio, USA**
**STANISLAW BILINSKI et al. « Synthesis of some derivatives of isonicotinic acid »**
**CHEMICAL ABSTRACTS, vol. 54, n° 5, 10 mars 1960, Columbus, Ohio, USA**
**FUMIO UEDA et al. « Syntheses and antimicrobial activity of 2-amino-4-phenylthiazole derivatives »**
**CHEMICAL ABSTRACTS, vol. 60, n° 2, 20 janvier 1964, Columbus, Ohio, USA**
**ZUGRAVESCU et al. « Thiazole derivatives. V. Thiazolylcarbamates and thiazolylsemicarbazides »**
**CHEMICAL ABSTRACTS, vol. 63, n° 11, 22 novembre 1965, Columbus, Ohio, USA**
**EUGENIA CARP et al. « Thiazole derivatives and study of their antituberculous actions »**
**CHEMICAL ABSTRACTS, vol. 79, n° 17, 29 octobre 1973, 101522h, Columbus, Ohio, USA**
**H. TRIPATHY et al. « Search for new fungicides. I. Synthesis of some new halogenated N-thiazolyl-substituted hydroxy acid amides and their use as possible fungicides »**
**CHEMICAL ABSTRACTS, vol. 88, n° 23, 5 juin 1978, 165278b, Columbus, Ohio, USA**
**M. BANERJEE & B.C. DASH « Synthesis of some N-thiazolyl nicotinic acid amides and their uses as possible fungicides »**

(73) Titulaire : **PIERRE FABRE S.A.**
**125, rue de la Faisanderie**
**F-75116 Paris (FR)**

(72) Inventeur : **Tarayre, Jean-Pierre**
**Rue des Sports Valdurenque**
**F-81100 Castres (FR)**
Inventeur : **Cousse, Henri**
**Foun de los Noblos Chemin de Lastinos**
**F-81100 Castres (FR)**
Inventeur : **Mouzin, Gilbert**
**21, rue Sainte-Foy**
**F-81100 Castres (FR)**
Inventeur : **Lauressergues, Henri**
**Plateau Saint-Jean**
**F-81100 Castres (FR)**
Inventeur : **Casadio, Silvano**
**Via Tantardini, 15**
**I-20136 Milan (IT)**

(74) Mandataire : **Doat, Jean Pierre**
**17, Avenue Jean Moulin**
**F-81106 Castres Cedex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 17, rue du Louvre, 75001 Paris, France

EP 0 001 727 B1

Nouveaux dérivés du phényl-4 (trichloro-2'-2'-2', éthoxycarboxamido)-2 thiazole utiles comme médicaments, compositions pharmaceutiques les contenant et leur procédé de préparation

La présente invention, réalisée au Centre de Recherches Pierre FABRE, concerne de nouveaux dérivés de phényl-4 (trichloro-2'-2'-2' éthoxycarboxamido)-2 thiazole, leur procédé de préparation et leur application en tant que médicament utile dans le traitement de l'arthrite rhumatoïde. L'invention vise également les compositions pharmaceutiques contenant ces principes actifs.

Les nouveaux composés chimiques, objet de l'invention, sont des dérivés du phényl-4 (trichloro-2'-2'-2' éthoxycarboxamido)-2 thiazole de formule générale (I) :

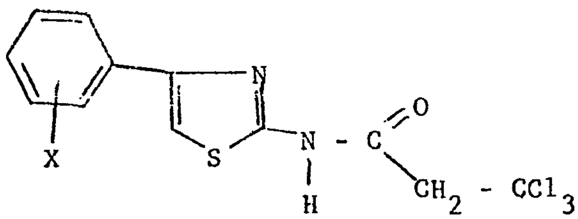

dans laquelle :

X qui se trouve en position 2, 3 ou 4 représente un atome d'hydrogène, un halogène, un groupe méthyle ou méthoxy.

Les composés de formule générale (I) peuvent être préparés en deux étapes par cyclisation d'un dérivé de l'acétophénone avec la thiourée, on obtient les amino-2 phényl-4 thiazoles, qui traités par le chloroformiate d'α,α,α-trichloroéthyle aboutissent aux composés (I) selon le schéma réactionnel :

1ère étape :

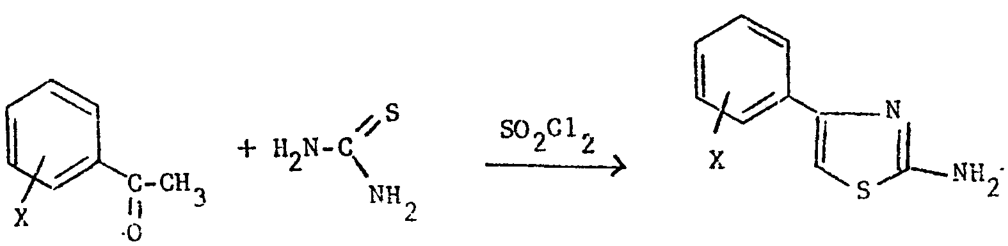


2ème étape :

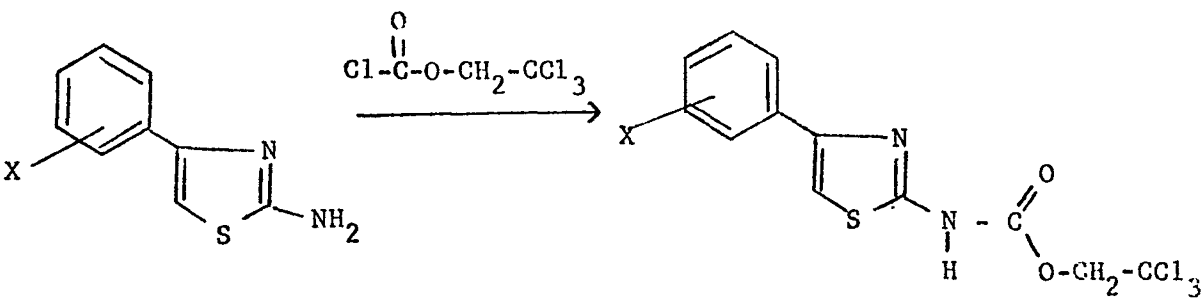

Les composés chimiques suivants et leur mode de préparation sont décrits à titre d'exemples non limitatifs.

Les exemples 1 à 4 décrivent la préparation d'intermédiaires de synthèse pour l'obtention des composés de l'invention.

Exemple 1 :

Phényl-4 amino-2 thiazole

Mélanger soigneusement 360 g (3 moles) d'acétophénone et 455 g (6 moles) de thiourée, puis ajouter par petites fractions 264 ml (3,3 moles) de chlorure de sulfuryle. La réaction est exothermique et l'addition du chlorure de sulfuryle dure 2 heures.

Le milieu réactionnel se liquéfie puis se prend en masse, porter alors à 105 °C pendant 3 heures.

Laisser revenir à température ambiante, laver à l'acétone puis filtrer. Les cristaux obtenus sont recristallisés dans 3 litres d'eau bouillante. On récupère le chlorhydrate de l'amine, qui est traité par une

solution d'ammoniaque jusqu'à pH 12.

On récupère après filtration et séchage 412 g de base.

Rendement 78 %.

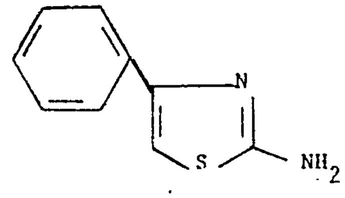

Formule brute : $C_9H_8N_2S$
Masse moléculaire : 176,24
Cristaux blancs
Point de fusion : 150 °C
Chromatographie en couche mince :
— support : gel de silice 60 F 254 Merck
— solvant : acide acétique - dioxanne - benzène - 2 - 8 - 90
— révélation : UV et iodé
— Rf : 0,34
Solubilités : insoluble dans l'eau, soluble à 1 % dans l'éthanol et à 20 % dans la diméthyl pyrrolidone.

Exemple 2 :

Para chloro phényl-4 amino-2 thiazole

D'une manière analogue à celle décrite dans l'exemple 1, mais en utilisant la parachloroacétophénone, on obtient le produit de formule :

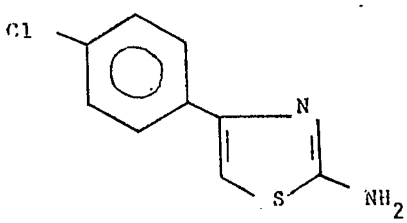

Formule brute : $C_9H_7ClN_2S$
Masse moléculaire : 210,67
Cristaux : jaunes
Point de fusion : 166 à 167 °C
Chromatographie en couche mince :
— support : gel de silice 60 F 254 Merck
— solvant : acide acétique - dioxanne - benzène - 2 - 8 - 90
— révélation : UV et iode
— Rf : 0,3
Solubilités : insoluble dans l'eau, soluble à 15 % dans l'éthanol.

Exemple 3 :

Para méthyl phényl-4 amino-2 thiazole

D'une manière analogue à celle décrite dans l'exemple 1, mais en utilisant la paraméthylacétophénone, on obtient le produit de formule :

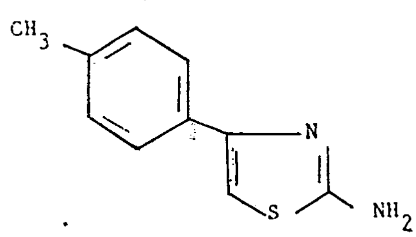

Exemple 4 :

Para méthoxy phényl-4 amino-2 thiazole

D'une manière analogue à celle décrite dans l'exemple 1, mais en utilisant la para méthoxy acétophénone, on obtient le produit de formule :

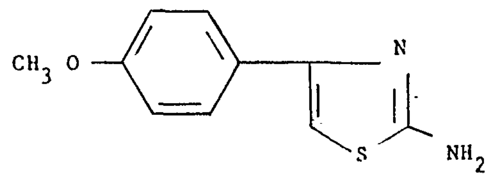

Exemple 5 :

Phényl-4 (trichloro-2'-2'-2', éthoxycarboxamido)-2 thiazole

A une solution de 35,2 g de phényl-4 amino-2 thiazole dans 150 ml d'acétone, ajouter 20 g de bicarbonate.

Puis, sous forte agitation, additionner lentement 42 g de chloroformate de ααα trichloroéthyle.

Maintenir l'agitation une nuit à température ambiante.

Filtrer et évaporer jusqu'à siccité, la masse résiduelle est recristallisée dans un mélange alcool-eau 50/50. On récupère 32 g (55 %) de produit de formule :

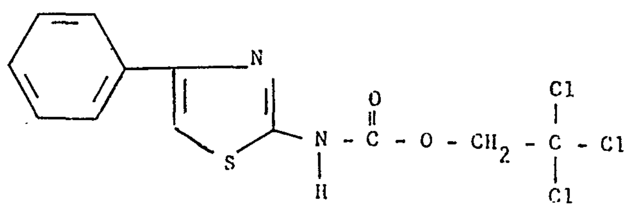

Formule brute : $C_{12}H_9Cl_3N_2O_2S$
Masse moléculaire : 351,64
Cristaux : blancs
Point de fusion : 126 °C
Chromatographie en couche mince :
— support : gel de silice 60 F 254 Merck
— solvant : acide acétique - dioxanne - benzène - 02 - 08 - 90
— révélation : UV et iode
— Rf : 0,61

Solubilités : insoluble dans l'eau, soluble à 3 % dans l'éthanol, à 25 % dans le DMA et à 20 % dans la méthyl pyrrolidone.

Exemple 6 :

Parachlorophényl-4 (trichloro-2'-2'-2' éthoxycarbamido)-2 thiazole

D'une manière analogue à celle décrite dans l'exemple 11, mais en utilisant le parachlorophényl-4 amino-2 thiazole et le chloroformiate de ααα trichloroéthyle, on obtient le produit de formule :

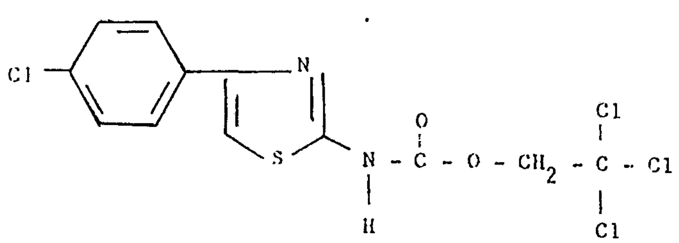

Formule brute : $C_{12}H_{18}Cl_4N_2O_2S$
Masse moléculaire : 386,08
Cristaux : beiges

Point de fusion : 200 °C
Chromatographie en couche mince :
— support : gel de silice 60 F 254 Merck
— solvant : acide acétique - dioxanne - benzène - 02 - 08 - 90
— révélation : UV et iode
— Rf : 0,75
Solubilités : insoluble dans l'eau, soluble à 20 % dans le DMA et dans la méthyl pyrrolidone.

## EXPÉRIMENTATIONS :

Les composés chimiques précédemment décrits ont fait l'objet d'expérimentations pharmacologiques et toxicologiques en vue de déterminer les possibilités d'application dans le domaine thérapeutique.

## A) PHARMACOLOGIE :

I — Mise en évidence d'une activité immunomodulatrice :

1) Technique :

Réaction d'hypersensibilité retardée cutanée à la tuberculine chez le cobaye albinos.
(Technique de G. L. Floersheim — Helv. Physiol. Acta II, 92, 1964, modifiée).

a) Sensibilisation :

On utilise des Mycobacterium tuberculosis tués. On prépare une suspension bien homogénéisée de 80 mg de Mycobacterium tuberculosis (passés préalablement au mortier) dans 10 ml d'adjuvant incomplet de Freund + 10 ml de tampon phosphate 0,2 M (pH ≃ 7).

On injecte 0,5 ml de cette suspension par voie intra-veineuse dans une cuisse du cobaye.

b) Challenge :

A différents temps après la sensibilisation, on injecte par voie intradermique l'antigène purifié (PPD) à raison de 5 mcg par injection sur les flancs rasés du cobaye.

On confectionne ainsi 4 papules par flanc. 24 heures après, on observe au niveau des injections des érythèmes de forme elliptique. On mesure deux diamètres perpendiculaires de chaque papule afin d'en calculer la surface.

c) Traitement par les produits à étudier :

Les produits sont administrés autour de la période de l'injection challenge, selon les modalités suivantes : les produits sont administrés par voie sous cutanée, dissous dans du diméthyl sulfoxyde (DMSO) à 3 reprises : 24 heures et 2 heures avant les injections de PPD, la dernière administration est pratiquée 2 heures après celles-ci.

2) Résultats :

Le tableau n° 1 rapporte les résultats obtenus avec le produit de l'exemple 5 et le lévamisole (L-tétramisole).

Chez les animaux témoins, la réponse d'hypersensibilité cutanée retardée au PPD est d'autant plus importante que l'intervalle entre la sensibilisation et le challenge est plus grand. (Des résultats non rapportés ici montrent qu'au delà de 6 semaines la réponse demeure constante).

Administrés autour de la période du challenge, le produit de l'exemple 5 et, à un degré nettement inférieur, le lévamisole, stimulent la réponse lorsque celle-ci est faible en intensité chez les témoins (c'est-à-dire lorsque l'intervalle entre la sensibilisation et le challenge est de 2 et 3 semaines), alors qu'ils ne la modifient plus de manière significative lorsqu'elle atteint une certaine intensité (après 5 à 6 semaines).

3) Conclusions :

Ces résultats illustrent le concept d'agents immunomodulateurs : ces produits, dans certaines conditions d'administration (ici autour de la période du challenge) restaurent la réponse immunitaire (essentiellement l'immunité cellulaire) lorsque celle-ci est déprimée. Lorsque la réponse est normale ils n'ont plus d'action significative. Dans le cadre de l'expérience, le produit de l'exemple 5 ne stimule la réaction au PPD que lorsque les cobayes sont en état d'hypo-immunisation.

II — Action sur la pleurésie d'hypersensibilité retardée à Bordetella pertussis chez le rat après un traitement chronique :

1) Technique :

Inspirée de : — Dieppe P. A., Willoughby D. A., Huskisson E. C., Arrigoni-Martelli E. — Agents and actions, 6, 618, 1976.
— Tarayre J. P., Delhon A., Lauressergues H., A Inst. Pharmacodyn. 228, 162, 1977.

a) Sensibilisation :

On utilise des rats males pesant 280-320 g. La suspension de Bordetella pertussis employée est le vaccin anti-coquelucheux de l'Institut Pasteur ($5 \times 10^9$) germes tués par ml. La suspension à Bordetella pertussis est mélangée avec de l'adjuvant complet de Freund. 0,2 ml de ce mélange est injecté par voie intramusculaire dans chaque cuisse du rat.

b) Challenge :

6 jours plus tard, 0,1 ml de la suspension à Bordetella pertussis est injecté dans la cavité pleurale. 48 heures plus tard, l'exsudat pleural formé est prélevé et mesuré. On détermine le nombre total de leucocytes, le nombre de mononucléaires et de polynucléaires de l'exsudat.

c) Traitement :

Les produits étudiés ont été administrés en chronique selon deux modes :

Modalité 1 : Le traitement débute le jour de la sensibilisation et se poursuit à raison d'une administration par jour jusqu'à l'injection challenge. Le jour de celle-ci, les composés sont donnés deux fois : une administration avant et une après l'injection intrapleurale de l'antigène. Les animaux sont traités une dernière fois 24 heures après.

Modalité 2 : Le traitement débute 3 semaines avant la sensibilisation. Durant ces 3 semaines les produits sont donnés à raison de 5 fois par semaine. Le traitement est ensuite continué comme indiqué ci-dessus dans la modalité 1.

2) Résultats :

Ils sont rapportés dans le tableau 2.

Administrés par voie orale en traitement chronique, à la dose de 5 mg.kg/administration, les produits de l'exemple 5 réduisent l'inflammation pleurale d'hypersensibilité retardée.

Son action est supérieure à celle du lévamisole et apparaît au cours des deux modes de traitement mis en jeu.

L'effet du produit porte sur le volume de l'exsudat et également sur les phénomènes cellulaires de la réaction.

B) TOXICOLOGIE :

I — Toxicité aiguë :

1) Technique :

La toxicité aiguë après une seule administration des produits par voie orale a été étudiée chez la souris.

Les composés sont mis en suspension dans un mélange tween 80 et eau distillée. La mortalité est relevée au bout de 7 jours.

Pour les produits présentant une mortalité à 1 g/kg où à des doses inférieures, la DL 50 (dose entraînant 50 % de mortalité) a été déterminée selon la technique de Miller C. et Tainter M. L. - Proc. Soc. Exp. Biol.

II — Toxicité chronique

Administré par voie orale pendant deux mois chez le rat, le produit de l'exemple 5 n'a entraîné aucun phénomène toxique jusqu'à la dose de 30 mg/kg/jour.

TABLEAU 1

Action du produit de l'exemple 5 et du lévamisole, administrés autour de la période du challenge, sur la réaction cutanée au PPD chez le cobaye

| Période entre la sensibilisation et lechallenge | Lots | Paramètres | Surface del'érythème a + 24 h (enmm$^2$) |
|---|---|---|---|
| 2 semaines | Témoins DMSO1 ml/kg(× 3) | Moyenne ±erreur stan-dard | 3,4 ± 1,8(3,24) |
| | Exemple 5 5 mg/kg (× 3) | Moyenne ± erreurstandard Actionsignificative | 29,8 ± 6,5 (3,24) + 776 % $p < 0,01$ |
| | Lévamisole 5 mg/kg (× 3) | Idem | 6,8 ± 4,9(2,16) + 100 % $p > 0,05$ |
| 3 semaines | Témoins | | 54,4 ± 11,1(6,47) |
| | Exemple 5 5 mg/kg (× 3) | | 98,9 ± 8,6(7,55) + 82 % $p < 0,01$ |
| | Lévamisole 5 mg/kg (× 3) | | 72,4 ± 7,8(7,56) + 33 % $p > 0,05$ |

Tableau 1 (Suite)

| Période entre la sensibilisation et le challenge | Lots | Paramètres | Surface de l'érythème a + 24 h (en $mm^2$) |
|---|---|---|---|
| 5 semaines | Témoins | | 101,3 ± 9,2(3,20) |
| | Exemple 5<br>5 mg/kg(× 3) | | 99,3 ± 6,9(3,18)<br>– 2 %<br>$p > 0,005$ |
| | Lévamisole<br>5 mg/kg(× 3) | | 83,7 ± 5,0(3,24)<br>– 17 %<br>$p > 0,005$ |
| 6 semaines | Témoins | | 144,3 ± 7,7(5,37) |
| | Exemple 5<br>5 mg/kg(× 3) | | 140,1 ± 8,1(8,46)<br>– 3 %<br>$p > 0,05$ |

* Entre parenthèses, dans l'ordre : (nombre de cobayes, nombre de papules)

TABLEAU 2
Pleurésie d'hypersensibilité retardée à Bordetella pertussis chez le rat

| Produits | Mode de traitement* | Doses mg/kg | Voie | Action sur le volume d'exsudat | Action sur le nombre total de leucocytes | Action sur le nombre de mononucléaires | Action sur le nombre de polynucléaires |
|---|---|---|---|---|---|---|---|
| Exemple 5 | 1 | 5 | p.o. | – 35 %<br>(35)<br>$p < 0,05$ | – 19 %<br>$p < 0,05$ | – 21 %<br>$p < 0,05$ | – 16 %<br>$p > 0,05$ |
| Lévamisole | 1 | 5 | p.o. | – 22 %<br>(36)<br>$p > 0,05$ | – 10 %<br>$p > 0,05$ | – 11 %<br>$p > 0,05$ | – 9 %<br>$p > 0,05$ |
| Exemple 5 | 2 | 5 | p.o. | – 50 %<br>(29)<br>$p < 0,05$ | – 22 %<br>$p < 0,05$ | – 29 %<br>$p < 0,01$ | – 6 %<br>$p > 0,05$ |
| | 2 | 5 | p.o. | – 42 %<br>(21)<br>$p < 0,01$ | | | |
| Lévamisole | 2 | 5 | p.o. | – 23 %<br>(29)<br>$p < 0,05$ | – 13 %<br>$p < 0,01$ | – 17 %<br>$p < 0,01$ | – 7 %<br>$p > 0,05$ |

* Mode de traitement 1 : traitement débutant le jour de la sensibilisation
Mode de traitement 2 : traitement débutant 3 semaines avant la sensibilisation

## APPLICATIONS THÉRAPEUTIQUES :

Compte tenu des propriétés pharmacologiques de ces composés chimiques, ils peuvent être utilisés dans les thérapeutiques nécessitant l'utilisation d'immunomodulateurs, par exemple dans le traitement d'affections respiratoires, de la polyarthrite rhumatoïde, ou en cancérologie. Ils sont utiles également en thérapeutique vétérinaire, par exemple dans les élevages d'animaux sacrifiés jeunes où l'on ne procède pas à des vaccinations, d'où l'intérêt d'un traitement par immunomodulateurs.

**Revendications**

1. Nouveaux dérivés du phényl-4 (trichloro-2'-2'-2', éthoxycarboxamido-2 thiazole répondant à la formule générale (I) :

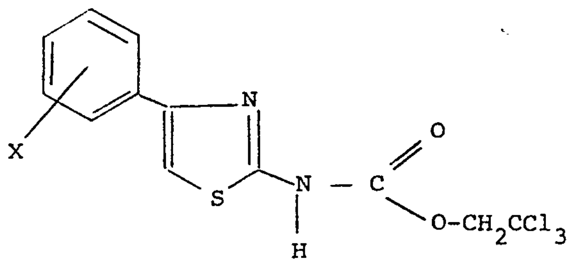

(I)

dans laquelle :

X qui se trouve en position 2, 3 ou 4 représente un atome d'hydrogène, un halogène, un groupe méthyle ou méthoxy.

2. Composé de formule générale (I) selon la revendication 1 et plus particulièrement : le phényl-4 (trichloro-2'-2'-2' éthoxycarboxamido)-2 thiazole.

3. Composé de formule générale (I) selon la revendication 1 et plus particulièrement : le para chloro phényl-4 (trichloro-2'-2'-2' éthoxycarboxamido)-2 thiazole.

4. Procédé de préparation des composés de formule générale (I) selon les revendications 1 à 3, caractérisé par le fait qu'il consiste à faire réagir un dérivé du phényl-4 amino-2 thiazole de formule générale (II) :

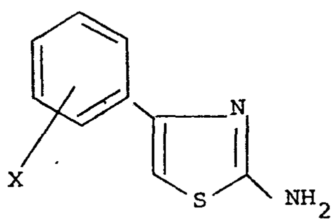

(II)

avec le chloroformiate d'α,α,α-trichloroéthyle.

X a la signification donnée dans la revendication 1.

5. A titre de médicaments nouveaux les composés chimiques selon l'une des revendications 1 à 3.

6. Les compositions pharmaceutiques caractérisées par le fait qu'elles contiennent à titre de substance active au moins un composé selon les revendications 1 à 3 seul ou associé à d'autres principes actifs.

**Claims**

1. New derivatives of 4-phenyl-2-(2',2',2',-trichloroethoxycarboxamido)-thiazole corresponding to the general formula (I) :

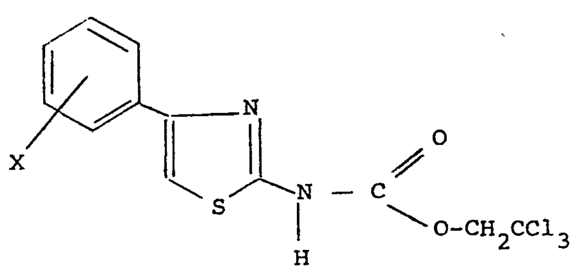

(I)

in which :

X, which is in the 2-, 3- or 4-position, represents a hydrogen atom, a halogen atom, a methyl group or a methoxy group.

2. Compound of the general formula (I) according to claim 1 and in particular : 4-phenyl-2-(2',2',2'-trichloroethoxycarboxamido)-thiazole.

3. Compound of the general formula (I) according to claim 1 and in particular : 4-p-chlorophenyl-2-(2',2',2'-trichloroethoxycarboxamido)-thiazole.

4. Process for the preparation of compounds of the general formula (I) according to claims 1 to 3, characterised in that it consists in reacting a derivative of 2-amino-4-phenylthiazole of the general formula (II) :

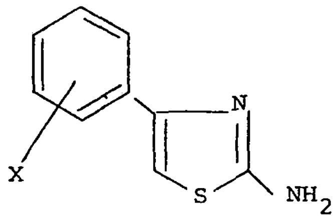

(II)

with α,α,α-trichloroethyl chloroformate, X having the meaning given in claim 1.

5. The chemical compounds according to one of claims 1 to 3 as new medicaments.

6. Pharmaceutical compositions characterised in that they contain as the active substance at least one compound according to claims 1 to 3 alone or in conjunction with other active ingredients.

**Ansprüche**

1. Neue Derivate von 4-Phenyl-2(2',2',2'-trichlorethoxycarboxamido) thiazol gemäß der allgemeinen Formel (I) :

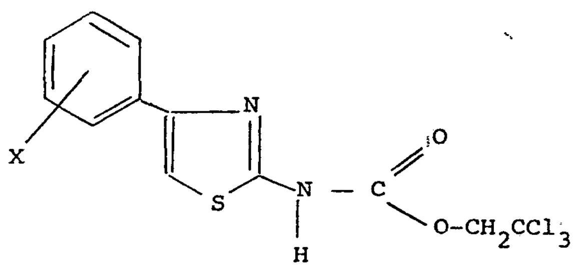

(I)

in der :

X, welches in Stellung 2, 3 oder 4 stehen kann, ein Wasserstoffatom, ein Halogenatom, eine Methyl- oder Methoxygruppe bedeutet.

2. Verbindung der allgemeinen Formel (I) gemäß Anspruch 1, insbesondere das 4-Phenyl-2(2',2',2'-trichlorethoxycarboxamido) thiazol.

3. Verbindung der allgemeinen Formel (I) gemäß Anspruch 1, insbesondere das 4-Parachlorphenyl-2 (2',2',2'-trichlorethoxycarboxamido) thiazol.

4. Verfahren zur Herstellung von Verbindungen der allgemeinen Formel (I) gemäß den Ansprüchen 1 bis 3, dadurch gekennzeichnet, daß man ein Derivat des 4-Phenyl-2-amino-thiazols der allgemeinen Formel (II).

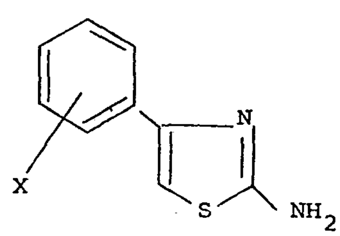

(II)

in der X die in Anspruch 1 angegebene Bedeutung aufweist, mit α,α,α-Trichlorethyl Chlorformiat umsetzt.

5. Die chemischen Verbindungen nach einem der Ansprüche 1 bis 3 als neue Arzneimittel.

6. Pharmazeutische Zusammensetzung, dadurch gekennzeichnet, daß sie als Wirkstoff mindestens eine Verbindung nach den Ansprüchen 1 bis 3 allein oder zusammen mit anderen Wirkstoffen enthält.